(19) Europäisches Patentamt
European Patent Office
Office européen des brevets

(11) **EP 2 205 144 B1**

(12) **FASCICULE DE BREVET EUROPEEN**

(45) Date de publication et mention
de la délivrance du brevet:
**15.08.2012 Bulletin 2012/33**

(21) Numéro de dépôt: **08842948.5**

(22) Date de dépôt: **01.10.2008**

(51) Int Cl.:
***A61B 3/11*** *(2006.01)*

(86) Numéro de dépôt international:
**PCT/FR2008/051766**

(87) Numéro de publication internationale:
**WO 2009/053570 (30.04.2009 Gazette 2009/18)**

(54) **PUPILLOMETRE AUTOMATIQUE A VERIFICATION VISUELLE**

AUTOMATISCHES PUPILLOMETER MIT SICHTPRÜFUNG

AUTOMATIC PUPILOMETER WITH VISUAL CHECK

(84) Etats contractants désignés:
**AT BE BG CH CY CZ DE DK EE ES FI FR GB GR HR HU IE IS IT LI LT LU LV MC MT NL NO PL PT RO SE SI SK TR**

(30) Priorité: **09.10.2007 FR 0758167**

(43) Date de publication de la demande:
**14.07.2010 Bulletin 2010/28**

(73) Titulaire: **Essilor International (Compagnie Générale d'Optique)**
**94220 Charenton-le-Pont (FR)**

(72) Inventeur: **NAUCHE, Michel**
**F-94220 Charenton Le Pont (FR)**

(74) Mandataire: **Lenne, Laurence**
**Feray Lenne Conseil**
**Le Centralis**
**63, avenue du Général Leclerc**
**92340 Bourg-la-Reine (FR)**

(56) Documents cités:
**EP-A- 0 395 478    DE-A1- 10 300 188**
**FR-A- 2 538 239    FR-A- 2 618 666**
**US-A- 5 680 196**

**Description**

L'invention concerne un pupillomètre automatique à vérification visuelle.

Un pupillomètre est un appareil destiné à la mesure de L'écart pupillaire d'un individu. Il consiste à mettre en oeuvre une source lumineuse propre à la génération d'un reflet cornéen sur au moins l'un des yeux de L'individu et à procéder à La localisation de ce reflet.

Un tel pupillomètre peut être manuel comme par exemple décrit dans le document de brevet FR 1 506 352.

L'appareil est alors pourvu de trois fenêtres, deux fenêtres reproduisant une monture de Lunettes destinée à être posée sur le nez de L'individu et une fenêtre opposée où l'oeil d'un observateur responsable de La mesure est placé au foyer d'une lentille de collimation permettant de positionner la source lumineuse à L'infini pour correspondre à la vision de loin de L'individu. Cette lentille peut être translatable selon L'axe de visée, afin de permettre une mesure en vision de près ; elle est dans ce cas approchée de La source Lumineuse. L'observateur en position de mesure déplace un repère mobile et Le fait correspondre avec Le reflet cornéen pour obtenir la trace de L'axe de chaque pupille et La mesure du demi écart pupillaire de chaque oeil de L'individu.

Ce type de pupillomètre manuel est consommateur de temps et pose des problèmes de précision de mesure.

Plus récemment, il a été proposé des pupillomètres automatiques comme par exemple dans Le document de brevet FR 2 618 666.

Les rayons lumineux correspondant aux reflets cornéens y sont dirigés vers un récepteur de détection, qui est un récepteur photo sensible ou une photo diode, et la distance pupillaire est ensuite calculée électroniquement. Il n'est alors plus besoin d'observateur, La mesure étant totalement automatique.

Ce type de pupillomètre, qui est particulièrement précis car indépendant d'un opérateur, pose cependant les problèmes suivants.

L'individu dont on mesure L'écart pupillaire peut en effet avoir un comportement préjudiciable à une bonne mesure. Il peut par exemple fermer Les yeux, battre des paupières ou ne pas regarder dans La bonne direction, à savoir celle de la source Lumineuse. Il en résulte une fausse mesure du demi écart pupillaire, voire une fausse mesure de l'écart pupillaire, si L'individu ne regarde pas à l'infini. De plus les conditions d'éclairage lumineux ambiant peuvent perturber la mesure, donnant des valeurs imprécises.

Dans un pupillomètre automatique, il est actuellement impossible de détecter une telle erreur.

Il peut être logiquement envisagé de vérifier le bon positionnement des yeux de L'individu de façon également automatique, compte tenu de la direction prise par l'évolution de cette technique de mesure par pupillomètre.

Cependant, le contrôle des yeux ouverts n'est pas toujours réalisable facilement, car il est possible par exemple que le reflet cornéen soit partiellement visible, La photo étant prise au moment où La paupière bouge, et qu'on obtienne donc un résultat approximatif en terme de précision, mais inqualifiable comme faux.

Dans Le cas où Le contrôle est possible, il faut envisager des composants électroniques capables de réaliser de nombreux calculs afin d'analyser L'image. Ces composants sont en général assez coûteux, encombrants et consommateurs d'énergie électrique ce qui pose donc des difficultés pour un appareil portable.

L'invention résout ce problème de vérification du bon positionnement des yeux de L'individu dans un pupillomètre automatique.

Pour ce faire, L'invention propose un pupillomètre automatique comportant une source Lumineuse propre à la génération d'un reflet cornéen sur au moins l'un des yeux d'un individu disposés chacun en face d'une première et seconde fenêtre, une lentille de collimation permettant au moins de positionner La source Lumineuse pour correspondre à la vision de Loin de L'individu, un récepteur de détection destiné à La localisation automatique de ce reflet et un calculateur de L'écart pupillaire dudit individu, caractérisé en ce qu'il comporte également une fenêtre où l'oeil d'un observateur responsable de La mesure est destiné à être placé au voisinage du foyer de Ladite lentille, de façon à visualiser et vérifier Les conditions de mesure.

Selon un mode de réalisation préféré, après vérification L'observateur valide la mesure et pour ce faire, le pupillomètre comporte un moyen d'actionnement destiné à être actionné par Ledit observateur et à valider Ladite mesure.

Ledit récepteur de détection est, avantageusement, une caméra.

Il peut comporter au moins un miroir assurant le transfert dudit reflet cornéen vers ledit récepteur de détection.

Avantageusement, Le pupillomètre conforme à l'invention comporte un agencement d'occultation monoculaire.

Ledit agencement d'occultation peut comporter deux écrans à diaphragme commandé électriquement disposés respectivement en face de la première fenêtre et de la seconde fenêtre.

De préférence, ledit calculateur comporte un module de calcul de l'écart pupillaire dudit individu, en vision de près.

Ledit calculateur peut comporter un module de calcul du rayon de l'oeil dudit individu.

Ledit calculateur peut comporter un module de correction de Ladite mesure en fonction de La distance des yeux dudit individu et de ses verres de Lunettes.

Avantageusement, Ladite source Lumineuse est une source infra rouge.

Le pupillomètre peut comporter également une source lumineuse pour mesure en vision de près.

L'invention est décrite ci-après plus en détail à L'aide de figures ne représentant que des modes de réalisation préférés de L'invention.

La figure 1 est une vue de dessus d'un pupillomètre conforme à L'invention.
La figure 2 est une vue en coupe selon II-II de La figure 1.
La figure 3 est une vue en coupe selon III-III de La figure 1.
La figure 4 est une vue en coupe selon IV-IV de la figure 1.
La figure 5 est une vue illustrant l'ensemble du pupillomètre.
La figure 6 est une vue schématique illustrant La correction de la mesure en fonction de La distance des yeux d'un individu et de ses verres de Lunettes.
Les figures 7 et 8 sont des vues de configurations fonctionnelles de variantes de l'invention.

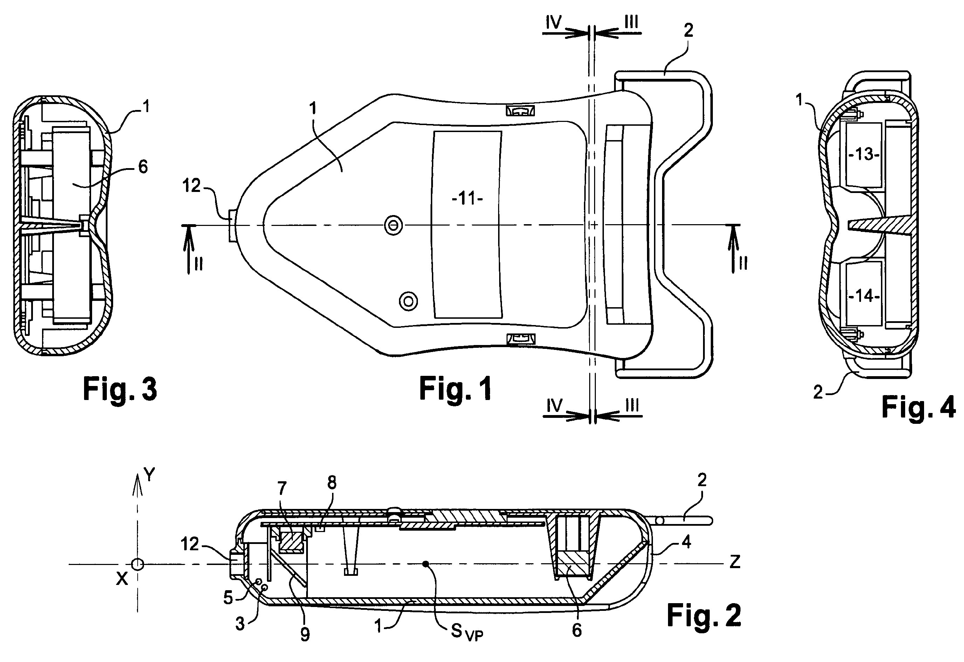

Un pupillomètre automatique conforme à L'invention est représenté en détail sur Les figures 1 à 4.

Ce pupillomètre comporte un boîtier 1 pourvu d'une partie 2 reproduisant une monture de Lunettes destinée à être posée sur Le nez d'un individu dont L'écart pupillaire est à mesurer.

A L'intérieur du boîtier, ce pupillomètre automatique comporte :

- une source Lumineuse infra rouge 3 propre à La génération d'un reflet cornéen sur au moins l'un des yeux de L'individu disposés chacun en face d'une première et seconde fenêtre 4,
- une source Lumineuse visible 5 disposée à côté de La source Lumineuse visible et destinée à être regardée et visée par L'individu,
- une lentille de collimation 6 permettant au moins de positionner La source Lumineuse 3, 5 pour correspondre à La vision de Loin de L'individu, la source Lumineuse infra rouge 3 et La source Lumineuse visible 5 étant disposées au voisinage du foyer de cette lentille 6,
- un récepteur de détection, constitué d'une caméra 7 destinée à La localisation automatique de ce reflet et
- un calculateur de L'écart pupillaire de L'individu porté par une carte électronique 8 contenue dans Le boîtier 1 et portant la caméra.

Compte tenu de La disposition de La caméra 7, un miroir semi transparent 9 incliné à 45° assure le transfert du flux Lumineux correspondant au reflet cornéen vers La caméra 7.

La carte électronique 8 comporte également un écran à diodes lumineuses ou un afficheur du type LCD 11 disposé devant une ouverture agencée dans le boîtier et assurant L'affichage des mesures de distance pupillaire et de demi écarts pupillaires.

En vis-à-vis des deux fenêtres 4 de regard et de visée de L'individu, Le boîtier comporte une troisième fenêtre 12 où l'oeil d'un observateur responsable de la mesure est destiné à être placé au foyer de la lentille de collimation 6, de façon à vérifier Les conditions de mesure.

Le pupillomètre comporte également un agencement d'occultation monoculaire, constitué de deux écrans 13, 14 à diaphragme commandé électriquement disposés respectivement en face de la première fenêtre et de La seconde fenêtre 4.

Le procédé de prise de mesure et Le fonctionnement d'un tel pupillomètre sont Les suivants.

L'observateur responsable de La mesure positionne le boîtier 1 du pupillomètre sur La visage de La personne, puis par regard dans La troisième fenêtre 12 vérifie visuellement que celle-ci regarde bien dans La bonne direction, à savoir vers La source Lumineuse visible 5, en observant que Les reflets cornéens sont bien centrés par rapport aux pupilles de l'oeil, et qu'il est dans une position de regard stable, à savoir qu'il a bien Les yeux ouvert. Quand ces conditions sont réunies, il Lance La mesure par appui sur un moyen d'actionnement de type bouton 13, ou valide la dernière mesure réalisée par appui sur ce même bouton 13, connecté électriquement au calculateur.

Fonctionnellement, Le regard de La personne vers La source Lumineuse visible 5, au travers des deux fenêtres 4, s'accompagne d'un regard quasi identique vers La source Lumineuse infrarouge 3. Cette source Lumineuse infra rouge 3 génère un reflet cornéen sur Les yeux de L'individu. Ce reflet cornéen est transmis en partie par réflexion sur Le miroir semi transparent 9 vers La caméra 7.

L'observateur observe Les reflets cornéens générés par La source Lumineuse visible 5.

On utilise une source infrarouge 3 en plus de La source visible 5 pour obtenir une meilleure image sur la caméra, car L'intensité de La réflexion sur La cornée peut être élevée sans éblouir La personne observée, et que L'impact du flux Lumineux parasite environnant est moindre. Il est possible néanmoins d'utiliser uniquement une source Lumineuse visible en déclenchant au moment de la validation de La mesure une augmentation brève de l'intensité Lumineuse de type flash Lumineux.

Dans le cas de l'utilisation d'une source infrarouge, on utilise préférentiellement un filtre infrarouge, le filtre étant adapté pour ne Laisser passer que les Longueurs d'onde correspondant à l'émission infrarouge de La source. Ce filtre est soit placé devant le capteur de La caméra ou devant La caméra, soit en alternative réalisé par le miroir 9, le miroir 9 réfléchissant alors uniquement L'émission infrarouge, les autres Longueurs d'onde le traversant. Ce type de miroir est appelé « miroir froid ».

Les données de localisation de La caméra sont transmises au calculateur qui peut être un calculateur basique, du type ADSP-BF531 de la société Analog Devices. La mesure s'effectue par calcul du barycentre des points d'intensité supérieur à un seuil, dans deux zones correspondant à l'oeil droit et à l'oeil gauche. Ce calcul nécessite très peu de puissance et peut être faite simultanément à la prise d'image par la caméra.

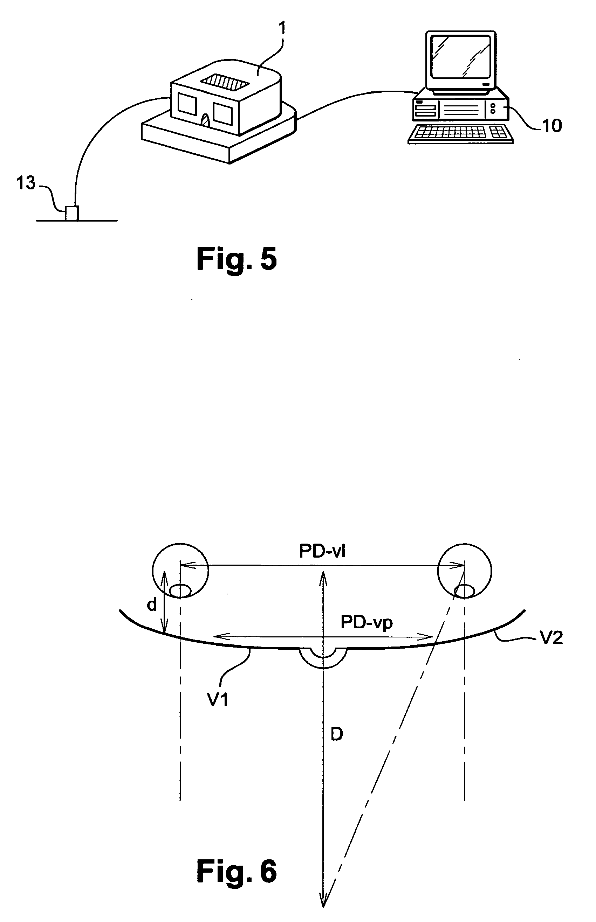

Le pupillomètre peut être disposé sur un socle, ce qui peut déclencher l'envoi automatique des mesures à un logiciel de traitement d'un ordinateur 10 et permettre La recharge des batteries, comme illustré sur la figure 5. La connexion entre le pupillomètre et L'ordinateur peut être également réalisée par réseau sans fil, du type WIFI ou équivalent.

Cette mesure peut être complétée ou remplacée par une mesure des demi écarts en monoculaire, au moyen des deux écrans 13, 14 à diaphragme commandé électriquement. Par réglage de La tension Les alimentant, de tels écrans peuvent passer de l'état transparent à l'état d'occultation et inversement, indépendamment l'un de L'autre. L'un des écrans peut donc être réglé à L'état d'occultation pour n'assurer une vue que de L'autre oeil par la personne.

On peut ainsi détecter des problèmes de strabisme, l'oeil ne focalisant pas sur la source lumineuse étant amené à bouger quand L'autre est occulté. On peut aussi détecter ainsi des problèmes d'amblyopie à savoir le cas d'un oeil qui ne voit pas du tout, car quand l'oeil normal est occulté, l'oeil amblyope donne un demi écart différent du demi écart mesuré en binoculaire.

Le calculateur comporte également un module de calcul de L'écart pupillaire de L'individu, en vision de près.

En effet, la mesure de la vision de près peut se déduire de La mesure de La vision de loin. Il suffit pour cela de connaître le rayon R de l'oeil, estimé égal au rayon moyen. On utilise la formule :

$$R = D \, (PDd\_vl - PDd\_vp) \, / \, PDd\_vl,$$

on a alors :

$$PDd\_vp = PDd\_vl - Rmoyen \times PDd\_vl/D$$

où PDd_vl est le demi écart pupillaire en vision de Loin,
PDd_vp est Le demi écart pupillaire en vision de près,
D est La distance entre la source Lumineuse 3 et l'oeil de l'individu en vision de près.

Le calculateur peut comporter également un module de correction de la mesure de distance pupillaire en fonction de la distance des yeux de L'individu et de ses verres de Lunettes. Si l'on considère La figure 6, la valeur de l'écart pupillaire en vision de près ramené dans le plan de La monture est directement impactée par la distance d entre le verre de Lunette $V_1$, $V_2$ et Le centre de rotation de l'oeil correspondant et cette distance n'est pas prise en compte dans Les pupillomètres actuels. Sur le pupillomètre conformément à L'invention, cette valeur de La distance d est saisie et une valeur de distance pupillaire en vision de près est calculée de la manière suivante :

$$PD\_vp = PD\_vl \; \times \; (D-d)/D$$

avec :

PD_vL distance pupillaire en vision de Loin,
PD-vp distance pupillaire en vision de près dans le plan de la monture,
D est la distance de présentation réelle ou théorique en vision de près.

Il est ainsi possible en ne mesurant que la vision de Loin, de déterminer précisément l'écart pupillaire en vision de près pour différentes valeurs de D , à condition de saisir La distance d entre le verre et le centre de l'oeil.

Des variantes peuvent être apportées à un tel pupillomètre tout en entrant dans le cadre de L'invention.

Selon le mode de réalisation décrit, Le trajet du rayon lumineux partant de La source lumineuse 3 vers l'oeil de la personne et celui partant du reflet cornéen vers la caméra 7 sont Légèrement décalés, du fait du décalage suivant la l'axe d'observation Z et L'axe vertical Y de la source lumineuse infra rouge 3 par rapport à la caméra. Le décalage suivant Z génère de légères imprécisions sur les mesures de demi-écart pupillaires que L'on peut corriger en utilisant par exemple une table de correction donnant la correction à réaliser sur chacun des demi-écarts pupillaire droit et gauche en fonction de leur valeur.

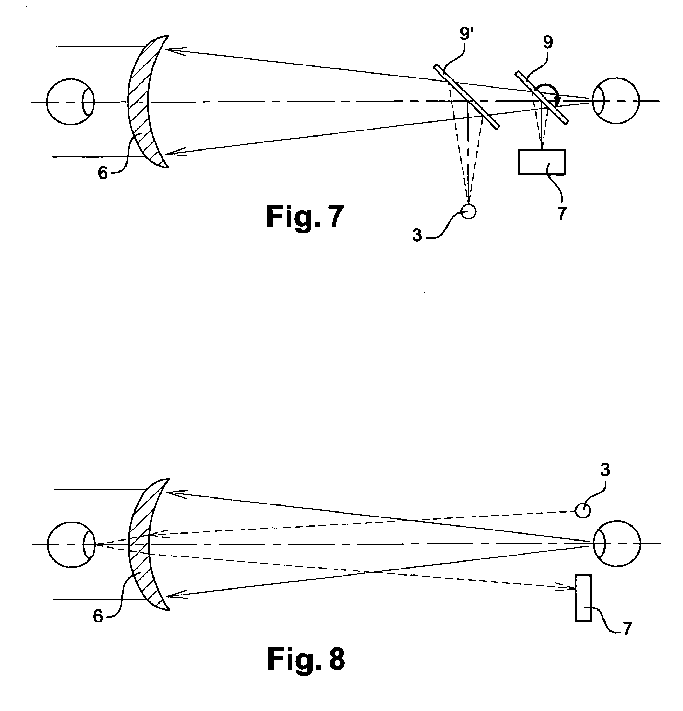

Ils peuvent être en grande partie confondus, en disposant plusieurs miroirs semi transparents, L'un 9' spécifique à la source Lumineuse et L'autre 9 spécifique à la caméra, comme illustré sur La figure 7.

Au lieu d'utiliser un miroir semi transparent, il est possible d'utiliser un miroir 9 rotatif amovible spécifique à La caméra, comme illustré par une flèche. Dans ce cas, ce miroir se rabat en synchronisation avec L'appui sur le bouton 13, afin de permettre à la caméra de recevoir le flux Lumineux, puis se remet de Lui-même à sa position initiale. Cette synchronisation peut être commandée électriquement ou mécaniquement. Cette solution permet d'obtenir un flux lumineux plus important qu'avec une solution à miroir semi transparente fixe.

Il est également possible d'utiliser « un miroir froid » ne réfléchissant que La Lumière infrarouge. Dans ce cas, la source infra rouge 3 est nécessairement placée en aval du miroir. Cette configuration permet d'obtenir un flux lumineux maximum en visible pour L'observateur et en infrarouge pour la caméra, car le flux Lumineux visible est totalement transmis par le miroir 9, alors que le flux infra rouge est totalement réfléchi vers La caméra. Ce miroir froid assure de plus La fonction de filtrage en Longueur d'onde, puisqu'uniquement les Longueurs d'onde correspondant à la source infra rouge 3 6 sont transmises à la caméra.

Il est également possible, comme illustré sur la figure 8, de décaler le trajet du rayon lumineux partant de la source lumineuse 3 vers l'oeil de la personne et celui partant du reflet cornéen vers La caméra 7, de telle sorte qu'aucun miroir semi transparent ne soit nécessaire. L'observateur responsable de La mesure observe cependant un décalage du reflet cornéen par rapport à la pupille de La personne.

Dans le cas d'un décalage vertical, comme illustré sur La figure 8, aucune correction de mesure n'est nécessaire.

Dans le cas d'un décalage horizontal, on peut prévoir une correction moyenne des demi-écarts mesurés, en ajoutant ou soustrayant des mesures obtenues une valeur constante prédéterminée.

Les utilisateurs concernés par un pupillomètre conforme à l'invention sont les opticiens, observateurs responsables de la mesure. L'appareil leur permet de mesurer les demi écarts pupillaire rapidement et précisément, l'appareil étant de plus d'un coût proche des appareil actuels.

La lentille de collimation 6 peut être montée de façon translatable afin de permettre une mesure directe de L'écart pupillaire en vision de près ou on peut ajouter une source Lumineuse adaptée pour réaliser le test en vision de près et située entre La lentille et son point focal, comme précisé par le point Svp sur la figure 2.

Dans ce cas, le calculateur peut comprendre un module de calcul du rayon de l'oeil et l'appareil peut de plus avantageusement être utilisé pour la monturisation.

Il est en effet possible de mesurer le rayon de l'oeil à partir des mesures de demi écarts en vision de près et en vision de loin. En effet, on a R = D x (PDd-vl - PDd_vp) / PDd_vl.

## Revendications

1. Pupillomètre automatique comportant une source lumineuse (3) propre à la génération d'un reflet cornéen sur au moins l'un des yeux d'un individu disposés chacun en face d'une première et seconde fenêtre (4), une lentille de collimation (6) permettant au moins de positionner la source lumineuse pour correspondre à la vision de loin de l'individu, un récepteur de détection (7) destiné à la localisation automatique de ce reflet et un calculateur de L'écart pupillaire dudit individu, **caractérisé en ce qu'**il comporte également une troisième fenêtre (12) où l'oeil d'un observateur responsable de la mesure est destiné à être placé au voisinage du foyer de ladite lentille (6), de façon à lui permettre de visualiser et vérifier les conditions de mesure.

2. Pupillomètre selon la revendication précédente, **ca-**ractérisé en ce qu'**il comporte un moyen d'actionnement (13) destiné à être actionné par ledit observateur et à valider ladite mesure.

3. Pupillomètre selon l'une des revendications précédentes, **caractérisé en ce que** ledit récepteur de détection (7) est une caméra.

4. Pupillomètre selon l'une des revendications précédentes, **caractérisé en ce qu'**il comporte au moins un miroir (9) assurant le transfert dudit reflet cornéen vers ledit récepteur de détection (7).

5. Pupillomètre selon l'une des revendications précédentes, **caractérisé en ce qu'**il comporte un agencement d'occultation monoculaire.

6. Pupillomètre selon la revendication précédente, **caractérisé en ce que** ledit agencement d'occultation comporte deux écrans (13, 14) à diaphragme commandé électriquement disposés respectivement en face de la première fenêtre et de la seconde fenêtre (4).

7. Pupillomètre selon l'une des revendications précédentes, **caractérisé en ce que** ledit calculateur comporte un module de calcul de l'écart pupillaire dudit individu, en vision de près.

8. Pupillomètre selon l'une des revendications 1 à 6, **caractérisé en ce que** ledit calculateur comporte un module de calcul du rayon de l'oeil dudit individu.

9. Pupillomètre selon l'une des revendications précédentes, **caractérisé en ce que** ledit calculateur comporte un module de correction de Ladite mesure en fonction de la distance des yeux dudit individu et de ses verres de lunettes.

10. Pupillomètre selon l'une des revendications précédentes, **caractérisé en ce que** Ladite source Lumineuse (3) est une source infra rouge.

11. Pupillomètre selon L'une des revendications précédentes, **caractérisé en ce que** qu'il comporte également une source lumineuse pour mesure en vision de près.

## Claims

1. An automatic pupillometer comprising a light source (3) capable of generating a corneal reflection on at least one of the eyes of an individual each positioned in front of a first and a second window (4), a collimation lens (6) at least making it possible to position the light source so as to correspond to the distance vision of the individual, a detection receiver (7) for automatically locating the reflection, and a calculator for calculating the pupil deviation of said individual, **characterized in that** it also includes a third window (12) in which the eye of an observer responsible for the measurement is to be placed in the vicinity of the focus of said lens (6) in order to allow it to display and check the measurement conditions.

2. The pupillometer according to the preceding claim, **characterized in that** it comprises an actuating means (13) intended to be actuated by said observer and validate said measurement.

3. The pupillometer according to one of the preceding claims, **characterized in that** said detection receiver (7) is a camera.

4. The pupillometer according to one of the preceding claims, **characterized in that** it comprises at least one mirror (9) ensuring the transfer of said corneal reflection toward said detection receiver (7).

5. The pupillometer according to one of the preceding claims, **characterized in that** it comprises a monocular occultation arrangement.

6. The pupillometer according to the preceding claim, **characterized in that** said occultation arrangement comprises two screens (13, 14) having electrically-controlled diaphragms that are disposed respectively facing the first and second windows (4).

7. The pupillometer according to one of the preceding claims, **characterized in that** said calculator comprises a module for calculating the pupillary distance of said individual in near vision.

8. The pupillometer according to one of claims 1 to 6, **characterized in that** said calculator includes a module for calculating the radius of the eye of said individual.

9. The pupillometer according to one of the preceding claims, **characterized in that** said calculator comprises a module for correcting said measurement as a function of the distances of the eyes of said individual from the corresponding eyeglass lenses.

10. The pupillometer according to one of the preceding claims, **characterized in that** said light source (3) is an infrared source.

11. The pupillometer according to one of the preceding claims, **characterized in that** said pupillometer also comprises a light source for near vision measurement.

**Patentansprüche**

1. Automatischer Pupillometer, umfassend eine eigene Lichtquelle (3) zur Erzeugung eines Hornhautreflexes auf mindestens einem der Augen eines Patienten, die jeweils gegenüber einem ersten und einem zweiten Fenster (4) angeordnet sind, wobei eine Kollimationslinse (6) mindestens ermöglicht, die Lichtquelle anzuordnen, um der Weitsicht des Patienten zu entsprechen, einen Nachweisempfänger (7), der zur automatischen Lokalisierung dieses Reflexes bestimmt ist, und einen Kalkulator des Pupillenabstands des Patienten, **dadurch gekennzeichnet, dass** er auch ein drittes Fenster (12) umfasst, in dem das Auge eines Beobachters, der für die Messung verantwortlich ist, dazu bestimmt ist, in der Nähe des Brennpunkts der Linse (6) angeordnet zu sein, um ihm zu ermöglichen, die Messbedingungen zu visualisieren und zu überprüfen.

2. Pupillometer nach dem vorhergehenden Anspruch, **dadurch gekennzeichnet, dass** er ein Betätigungsmittel (13) umfasst, das dazu bestimmt ist, von dem Beobachter betätigt zu werden und um die Messung zu validieren.

3. Pupillometer nach einem der vorhergehenden Ansprüche, **dadurch gekennzeichnet, dass** der Nachweisempfänger (7) eine Kamera ist.

4. Pupillometer nach einem der vorhergehenden Ansprüche, **dadurch gekennzeichnet, dass** er mindestens einen Spiegel (9) umfasst, der die Übertragung des Hornhautreflexes auf den Nachweisempfänger (7) sicherstellt.

5. Pupillometer nach einem der vorhergehenden Ansprüche, **dadurch gekennzeichnet, dass** er eine monokulare Abdeckungsanordnung umfasst.

6. Pupillometer nach einem der vorhergehenden Ansprüche, **dadurch gekennzeichnet, dass** die Abdeckungsanordnung zwei Schirme (13, 14) mit einem Diaphragma umfasst, das elektrisch gesteuert wird, die jeweils gegenüber dem ersten Fenster und dem zweiten Fensters (4) angeordnet sind.

7. Pupillometer nach einem der vorhergehenden Ansprüche, **dadurch gekennzeichnet, dass** der Kalkulator ein Modul zur Berechnung des Pupillenabstands des Patienten bei Nahsicht umfasst.

8. Pupillometer nach einem der Ansprüche 1 bis 6, **dadurch gekennzeichnet, dass** der Kalkulator ein Modul zur Berechnung des Augenstrahls des Patienten umfasst.

9. Pupillometer nach einem der vorhergehenden Ansprüche, **dadurch gekennzeichnet, dass** der Kalkulator ein Modul zur Korrektur der Messung je nach dem Abstand der Augen des Patienten und seiner Brillengläser umfasst.

10. Pupillometer nach einem der vorhergehenden Ansprüche, **dadurch gekennzeichnet, dass** die Lichtquelle (3) eine Infrarotquelle ist.

11. Pupillometer nach einem der vorhergehenden Ansprüche, **dadurch gekennzeichnet, dass** er auch eine Lichtquelle zur Messung bei Nahsicht umfasst.

**Fig. 3**

**Fig. 1**

**Fig. 4**

**Fig. 2**

EP 2 205 144 B1

# Fig. 5

# Fig. 6

**Fig. 7**

**Fig. 8**

**RÉFÉRENCES CITÉES DANS LA DESCRIPTION**

**Documents brevets cités dans la description**

- FR 1506352
- FR 2618666